# EUROPÄISCHE PATENTSCHRIFT

(11) **EP 1 532 237 B1**
(45) Veröffentlichungstag und Bekanntmachung des Hinweises auf die Patenterteilung: **02.03.2011**
(21) Anmeldenummer: 03790846.4
(22) Anmeldetag: 01.08.2003
(51) Int. Cl.: C12M 3/06

(54) **ZELLKULTUREINSATZ**
CELL CULTURE INSERT
INSERT DE CULTURE DE CELLULES

(30) Priorität: 30.08.2002 DE 10240787
(43) Veröffentlichungstag der Anmeldung: 25.05.2005
(73) Patentinhaber: Oxyphen AG, 8620 Wetzikon (CH)
(72) Erfinder: WEDELL, Gabriele, 01728 Goppeln (DE); MATTHES, Helmut, 01454 Grosserkmannsdorf (DE)
(74) Vertreter: Hanewinkel, Lorenz
(86) Internationale Anmeldenummer: PCT/EP2003/008527
(87) Internationale Veröffentlichungsnummer: WO 2004/020571

(56) Entgegenhaltungen:
- EP-A- 0 590 513
- WO-A-92/07063
- US-A- 4 871 674
- US-A- 5 534 227
- US-A- 5 578 492

## Beschreibung

Die Erfindung betrifft einen Zellkultureinsatz mit einer becherförmigen Einsatzwand mit einem Membranfilterboden und mit oben umfangsmäßig verteilten herausstehenden Tragearmen und mit seitlichen Abstandhaltern für eine vertikale und horizontale Orientierung in einem Well mit einer Nährflüssigkeit in einer Zellkulturplatte.

Zellkultureinsätze sollen, um mit ihnen bequem arbeiten zu können, eine große Einfüllöffnung besitzen. Auch der untere Querschnitt mit der Membran soll ausreichend groß sein, um einen guten Flüssigkeitsaustausch zwischen dem Becherinhalt und dem Wellinhalt zu erlauben. Auch ist es günstig, die Zellkultureinsätze in das Well einzuhängen, um an dem unteren Rand des Einsatzes keine das Zellwachstum störenden Füße zu haben. Neben der Zugänglichkeit des Zellkultureinsatzes durch die Einfüllöffnung ist auch die Zugänglichkeit des Wellbodens für Pipetten wichtig.

So sind Zellkultureinsätze in Becherform, beispielsweise aus der Patentschrift US 4,871,674 und US 5,578,492, bekannt, die in ein Well eingehängt sind und den Zugang zum Zellkultureinsatz und zum Well erlauben. Die hier beschriebenen Zelleinsätze sind symmetrisch aufgebaut und besitzen im eingehängten Zustand rund um die Einsätze nur kleine Beschickungsfenster für das Einführen von Pipetten in das Well.

In der Patentschrift US 4,871,674 ist beschrieben, dass der Zellkultureinsatz in eine obere Stellung gebracht werden kann, um das Beschickungsfenster zum Einführen einer Pipette zu vergrößern. Dabei liegt der Zellkultureinsatz an der Wand des Wells an, wodurch die Zellen im Well geschädigt werden.

Weiterhin ist aus der US 5,272,083 ein Zellkultureinsatz bekannt, der an einem oberen Flansch eine sich unter 45 Grad konisch bis zu einem zylindrischen Kulturraum verengende Tragarmanordnung trägt, wobei zwischen den Tragarmen Ausnehmungen sind, die eine Pipettenzugänglichkeit erbringen. Der Kulturraum ist dabei erheblich, auf ca. ¼ des Durchmessers des Aufnahmeraums, eingeengt.

Die bekannten Zellkultureinsätze sind entweder aus völlig farblosen transparentem Material oder aus dem gleichen einfarbigen Kunststoff hergestellt wie die Zellkulturplatte mit den Wells. Dadurch ergibt sich eine schlechte Unterscheidbarkeit zwischen dem Well und dem Zellkultureinsatz, womit das manuelle und das automatische Arbeiten erschwert ist.

Es ist Aufgabe der Erfindung, einen verbesserten Zellkultureinsatz mit ausreichend großer Membranfläche anzugeben, bei dem das Beschickungsfenster für das Well wesentlich größer ist und bei dem keine Bewegung des Einsatzes erforderlich ist, um Pipetten in das Beschickungsfenster einzuführen.

Gelöst wird diese Aufgabe dadurch, dass die Abstandshalter auf dem Umfang des Zellkultureinsatz so verteilt und zur Seite so unterschiedlich lang ausgebildet sind, dass sich ein großes Beschickungsfenster und mehrere kleinere Fenster ergeben.

Vorteilhafte Ausgestaltungen der Erfindung sind in den Unteransprüchen angegeben.

Der becherförmige Zellkultureinsatz hängt an Tragarmen auf dem oberen Rand des Wells und ist über unterschiedlich lange Abstandhalter asymmetrisch positioniert. Dabei bildet sich zwischen den längsten Abstandhaltern ein Beschickungsfenster aus, in das bequem Pipetten eingerührt werden können.

Drei Abstandhalter, die umfangsmäßig auf mehr als den halben Umfang verteilt sind, sind minimal erforderlich, um den Zellkultureinsatz in der Öffnung des Wells definiert zu positionieren. Das größte Beschickungsfenster ergibt sich, wenn einer der drei Abstandhalter kürzer als die beiden anderen ist.

Günstig in dieser Anordnung ist, dass der kürzeste Abstandhalter einen Mindestabstand zwischen dem Zellkultureinsatz und der Wand des Wells so bestimmt, dass kapillare Engräume von Zellen zwischen dem Zellkultureinsatz und der Wellwand vermieden werden, so dass keine Flüssigkeit dort aufsteigt.

Vorteilhaft sind ein kurzer Abstandhalter und zwei gleich lange Abstandsstege umfangsmäßig gleich verteilt am Becher angeordnet. Die Abstandstege, die beim Einführen des Zellkultureinsatzes in das Well eine asymmetrische Positionierung darin übernehmen, sind vorteilhafterweise nach unten verjüngt. Die Tragearme sorgen dafür, dass der Zellkultureinsatz in einer definierten Höhe im Well hängt. Der kürzeste Abstandhalter sorgt dafür, dass ein Mindestabstand zwischen den Wänden vom Zellkultureinsatz und dem Well eingehalten wird.

In einer besonders vorteilhaften Ausführung erbringt mindestens eine in die Einsatzwand obenendig zwischen den langen Abstandsstegen ausgebildete Wandausnehmung eine noch weiter verbesserte Pipettenzugänglichkeit. Die Tiefe der Ausnehmung beträgt beispielsweise 20% der Einsatzhöhe. Die Unterkante der Wandausnehmung weist einen ausreichenden Sicherheitsabstand zum Nährlösungspegel auf. Die Ausnehmungskante verläuft zur Vermeidung von kritischen Biegespannungen zu den Tragarmen hin abgerundet divergierend nach oben. Die Einsatzwand weist einen relativ großen Durchmesser im Kulturraum auf, da sie nur mit Ausformschrägen versehen ist, die außen etwa 1,5 Grad und innen etwa 3,3 Grad betragen. Der freie Membrandurchmesser ist dadurch größer als der Radius des Wells. Wenn zwischen allen Tragarmen Wandaüsnehmungen eingebracht sind, ist außer der Pipettenzugänglichkeit auch der Pinzettenzugriff zu den Tragarmen wesentlich verbessert.

Trotz der exzentrischen Aufhängung des Zellkultureinsatzes im Well bleibt für die automatische optische Auswertung der Zellkulturplatten mit den hier beschriebenen Zellkultureinsätzen ein genügend großes Beobachtungsfenster von z.B. 3 mm Durchmesser über der Mitte der Wells erhalten.

Wird ein größeres Beschickungsfenster benötigt, kann der Zellkultureinsatz mit einer Pipette nach oben geschoben werden, wobei es an den schrägen Abstandhaltern entlang ein Stück aus dem Well gleiten kann. Nach dem Entfernen der Pipette gleitet der Zellkultureinsatz an den Abstandhaltern entlang zuverlässig in das Well zurück.

Durch die hohe geschlossene Bauart des Zellkultureinsatzes wird eine Kontamination zwischen der Innen- und der Außenseite des Zellkultureinsatzes vermieden.

Für die Handhabung des Zellkultureinsatzes im Well lässt er sich mittels einer Pinzette an einem der langen Tragarme leicht erfassen, einsetzen und auch wieder herausheben.

Alle für Zellkultureinsätze bekannte Membranen können verwendet werden; Kapillarenmembranen aus Polyester oder Polycarbonat haben den Vorzug einer genau einstellbaren Porosität und Transparenz.

Vorteilhafterweise wird der Zellkultureinsatz aus einem eingefärbten Kunststoff hergestellt, der einen sichtbaren Kontrast zu dem Material des Wells darstellt. Damit ist die Erkennbarkeit der Einfüllöffnung verbessert. Vorzugsweise kommt ein transparenter farbiger Kunststoff zum Einsatz.

Eine Ausführung der Erfindung ist in den Figuren beispielhaft beschrieben.
Fig. 1 zeigt eine Draufsicht auf einen Zellkultureinsatz erster Ausführung im Well,
Fig. 2 zeigt eine Ansicht des Zellkultureinsatzes von der Seite mit drei Abstandstegen,
Fig. 3 zeigt eine Ansicht des Zellkultureinsatzes von der Seite mit Abstandstegen und Abstandshalter,
Fig. 4 zeigt eine Schnittdarstellung des Well mit Zellkultureinsatz mit Abstandhalter,
Fig. 5 zeigt eine Schnittdarstellung des Well mit Zellkultureinsatz mit schräger Wandung;
Fig. 6 zeigt eine zweite Ausführung des Einsatzes in Perspektive;
Fig. 7 zeigt dazu einen axialen Schnitt A - A;
Fig. 8 zeigt dazu eine Draufsicht.

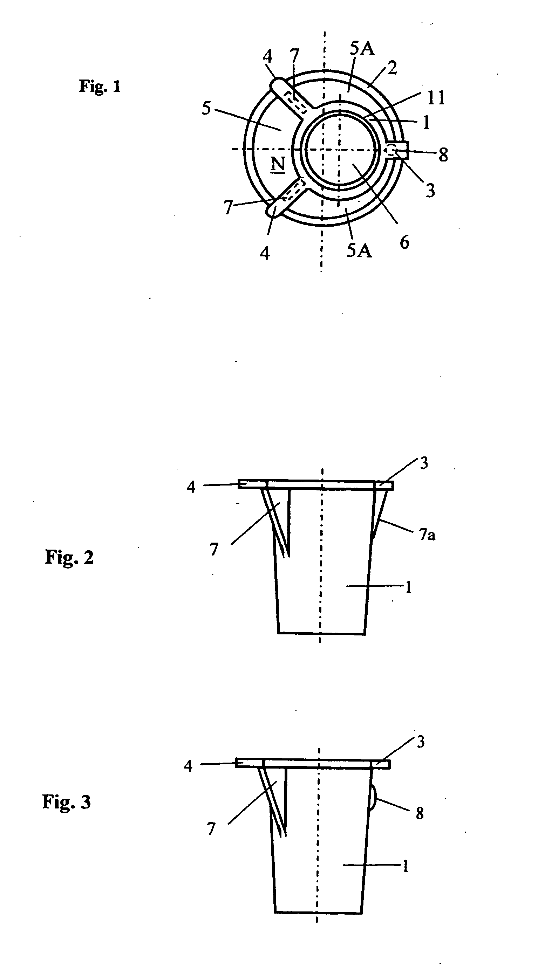

In Fig. 1 ist ein Zellkultureinsatz 1 erster Ausführung in der Draufsicht dargestellt, wie in einer runden Öffnung im Well 2 ein Zellkultureinsatz 1 außermittig zur Öffnung gehalten ist. Er wird von drei Tragearmen 3, 4 gehalten, wobei der Tragearm 3 kürzer als die beiden anderen ausgeführt ist. Dadurch ergibt sich ein großes Beschickungsfenster 5, in das eine Pipette leicht eingeführt werden kann, sowie zwei kleinere Fenster 5A. Auch die Öffnung 6 des Zellkultureinsatzes ist gut von oben zugänglich. Die Tragearme 3, 4 sind jeweils um 120 Grad versetzt angeordnet. Die Exzentrizität zwischen der Achse des Well und der des Einsatzes beträgt mehr als 1,3 mm, z.B. 4 mm. Diese wird durch unterschiedliche Abstandsstege, die unter den Tragarmen 3, 4 angeordnet sind, erreicht.

Fig. 2 zeigt eine Seitenansicht des becherförmigen Zellkultureinsatzes 1. An ihm sind lange dreieckförmigen Abstandstege 7 seitlich nach unten verjüngt und ein weiterer kürzerer Abstandsteg 7a angebracht. Oben sind an der Einsatzwand 11 die Tragearme 3, 4 ausgeformt, von denen der Tragearm 3 kürzer ist, der zu dem kürzeren Abstandssteg 7a gehört.

Fig. 3 zeigt eine Seitenansicht des becherförmigen Zellkultureinsatzes 1 in abgewandelter Form. An ihm sind die dreieckförmigen Abstandstege 7 und ein weiterer Abstandhalter 8 in Noppenform angebracht.

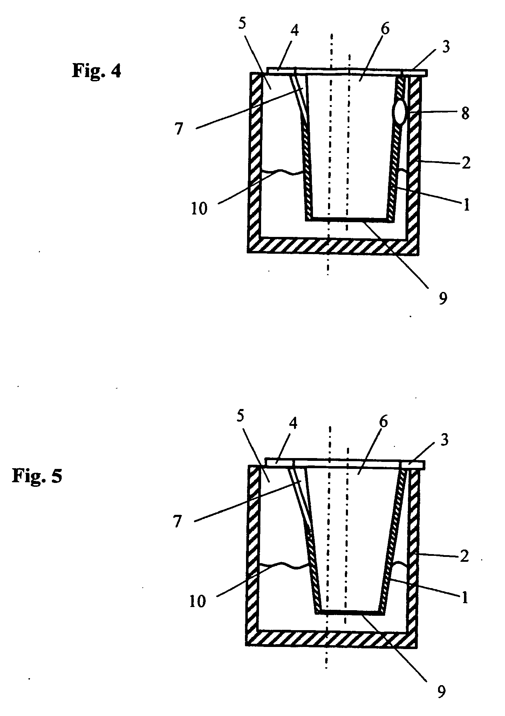

Fig. 4 zeigt einen Schnitt durch das zylindrische Well 2 mit eingehängtem Zellkultureinsatz 1. Die Tragearme 3, 4 liegen oben auf dem Well 2 auf und bestimmen die Höhenlage des Zellkultweinsatzes 1. Über die Abstandstege 7 und dem Abstandhalter 8 wird der Abstand zwischen den Wänden definiert gehalten. Das Beschickungsfenster 5 liegt zwischen den langen Tragearmen 4. Der Zellkultureinsatz 1 besitzt oben die Öffnung 6 und unten die Membran 9. Der Abstand zwischen den Wänden ist in der Pegelhöhe P des Flüssigkeitsspiegels 10 so groß, dass sich keine Kapillarwirkung ergibt.

Fig. 5 zeigt den gleichen Schnitt wie Fig. 4, mit dem Unterschied, dass der Abstand zwischen den Wänden des Einsatzes 1 und des Wells 2 in Höhe des Flüssigkeitsspiegels 10 durch die relativ größere Schräge des Zellkultureinsatzes 1 bestimmt wird, die im Beispiel ca. 7 Grad beträgt.

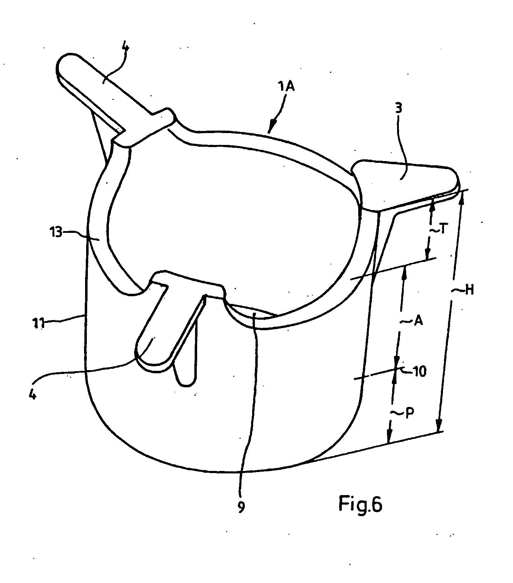

Fig. 6 zeigt perspektivisch eine weitere Ausführungsform eines Zellkultureinsatzes 1 A, bei dem die Einsatzwand 11 von oben zwischen den Tragarmen 3, 4 jeweils eine Wandausnehmung 12 aufweist, die zu den Tragarmen hin abgerundet ausgebildet ist. Die Ausnehmungstiefe T beträgt etwa 20% der Einsatzhöhe H, so dass die Unterkante 13 der Wandausnehmungen 12 einen ausreichenden Pegelabstand H zum üblichen Flüssigkeitsspiegel 10 aufweist.

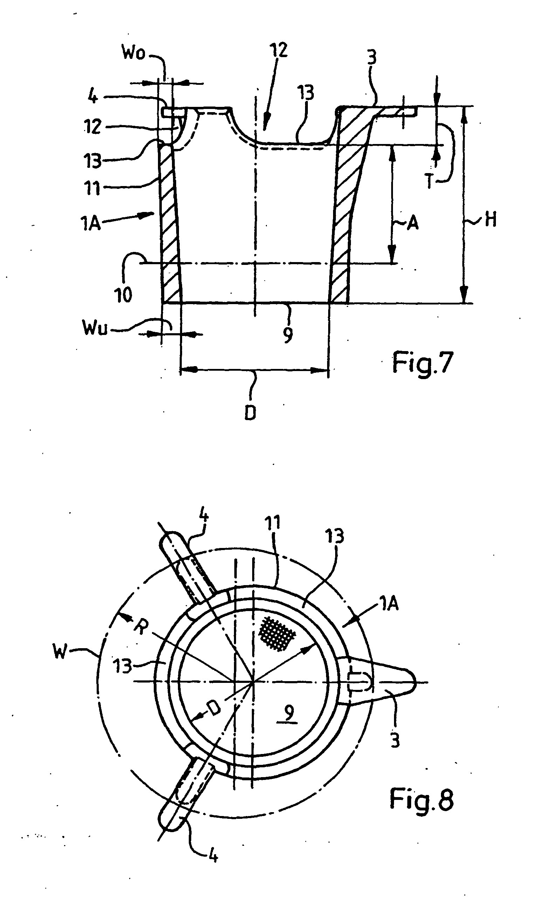

Fig.7 zeigt einen axialen Querschnitt durch die Ausführung Fig. 6. Man erkennt dort das Größenverhältnis der Ausnehmungstiefe T zur Einsatzhöhe H, die etwa fünf mal so groß ist. Auch der Normalpegelstand P der Nährflüssigkeit 10 ist dargestellt, der etwa 1/3 der Einsatzhöhe H über der Membran 9 liegt. Die Einsatzwand 11 ist außen mit einer Ausformschräge von ca. 1,5 Grad nach unten konisch verjüngt und innen mit etwa 3,3 Grad verjüngt, so dass die obere Wandstärke WO nach unten von 1 mm auf eine untere Wandstärke WU von 1,5 mm zunimmt, was unten einen sicheren Schweißrand für die Membran 9 und oben mehr umlaufenden Freiraum schafft.

Fig. 8 zeigt die Draufsicht zu Fig. 7. Strichpunktiert ist die Innenwand W des Wells angedeutet, deren Wellradius R kleiner als der Membrandurchmesser D ist. Die Exzentrizität des Einsatzes 1A zur Wellinnenwand W beträgt etwa 1,5 mm im Beispiel.

Beispielsweise beträgt der Wellradius R 10 - 12 mm und der nutzbare Durchmesser D der Membran 9 11 - 13 mm. Die Einsatzwand 11 ist oben etwa 15 - 17 mm weit. Die Abstandshalter 7, 8 erbringen durch ihre unterschiedliche seitliche Erstreckung eine Exzentrizität des Einsatzes 1A im Well 2 von 1,3 - 1,7 mm.

### Bezugszeichenliste

- 1: 1A Zellkultureinsatz
- 2: Well
- 3: kurzer Tragearm
- 4: langer Tragearm
- 5: Beschickungsfenster groß
- 5a: Beschickungsfenster klein
- 6: Öffnung des Zellkultureinsatzes
- 7: Langer Abstandsteg
- 7a: Kurzer Abstandsteg
- 8: Abstandhalter
- 9: Membran
- 10: Flüssigkeitsspiegel
- 11: Einsatzwand
- 12: Wandausnehmung
- 13: Unterkante der Wandausnehmung
- A: Pegelabstand zur Unterkante
- D: Membrandurchmesser
- H: Einsatzhöhe
- N: Nährflüssigkeit
- P: Pegelhöhe
- R: Wellradius
- T: Ausnehmungstiefe
- W: Wellinnenwand
- WO: obere Wandstärke
- WU: untere Wandstärke

## Patentansprüche

1. Zellkultureinsatz (1) mit einer becherförmigen Einsatzwand (11) mit einem Membranfilterboden (9), einem oberen Rand und mit daran umfangsmäßig verteilten herausstehenden Tragearmen (3, 4) und mit seitlichen Abstandhaltern (7, 8) für eine vertikale und horizontale Orientierung in einem Well (2) mit einer Nährflüssigkeit (N) in einer Zellkulturplatte, **dadurch gekennzeichnet, dass**
die Abstandshalter (7, 8) auf dem Umfang des Zellkultureinsatz (1) verteilt und zur Seite unterschiedlich lang ausgebildet sind, wobei ein Abstandshalter (8) der kürzeste ist, so dass sich bei einer Aufhängung im Well (2) ein großes Beschickungsfenster (5) und mehrere kleinere Fenster (5A) ergeben.

2. Zellkultureinsatz nach Anspruch 1, **dadurch gekennzeichnet, dass** mindestens zwei der Abstandhalter (7) als nach unten verjüngte dreieckige Abstandstege ausgeführt sind.

3. Zellkultureinsatz nach Anspruch 1, **dadurch gekennzeichnet, dass** der kürzeste Abstandhalter (8) einen Mindestabstand zwischen der Einsatzwand (11) des Zellkultureinsatzes (1) und Innenwand (W) des Wells (2) bestimmt, der ein kapillares Aufsteigen der Nährflüssigkeit (N) verhindert.

4. Zellkultureinsatz nach Anspruch 1, **dadurch gekennzeichnet, dass** der Abstand zwischen der Einsatzwand (11) des Zellkultureinsatz (1) und einer Innenwand (W) des Wells (2) durch eine nach unten verjüngte Form der Einsatzwand (11) bestimmt ist.

5. Zellkultureinsatz nach Anspruch 1, **dadurch gekennzeichnet, dass** der Zellkultureinsatz (1) an drei Tragearmen (3, 4) in das Well (2) exzentrisch eingehängt gehalten ist.

6. Zellkultureinsatz nach Anspruch 5, **dadurch gekennzeichnet, dass** die Tragarme (3,4) anschließend über den Abstandhaltern (7, 8) und umfangsmäßig je um 120 Grad versetzt angeordnet sind.

7. Zellkultureinsatz nach Anspruch 1, **dadurch gekennzeichnet, dass** die Einsatzwand (11) zwischen mindestens den zwei längeren Tragearmen (4) oben eine Wandausnehmung (12) mit einer Unterkante (13) aufweist, die beabstandet über einem normalen Pegelstand (10) der Nährstoffflüssigkeit (N) liegt.

8. Zellkultureinsatz nach Anspruch 7, **dadurch gekennzeichnet, dass** die Unterkante (13) der Wandausnehmung (12) zu den Tragarmen (3, 4) bogenformig verläuft.

9. Zellkultureinsatz nach Anspruch 7, **dadurch gekennzeichnet, dass** die Wandausnehmung eine Tiefe (T) hat, die etwa 20% einer Einsatzhöhe (H) des Einsatzes (1, 1A) beträgt.

10. Zellkultureinsatz nach Anspruch 1, **dadurch gekennzeichnet, dass** seine Einsatzwand (11) membranseitig eine größere Wandstärke (WU) aufweist als obenseitig.

11. Zellkultureinsatz nach Anspruch 10, **dadurch gekennzeichnet, dass** seine Einsatzwand (11) außen von oben nach unten verjüngt mit einer Ausformschräge von etwa 1,5 Grad ausgebildet ist und die Einsatzwand (11) innen eine Schräge von 3,3 Grad aufweist.

12. Zellkultureinsatz nach Anspruch 11, **dadurch gekennzeichnet, dass** die Membran (9) einen größeren Durchmesser (D) hat als einen inneren Wellradius (R) des umgebenden Wells (2).

13. Zellkultureinsatz nach Anspruch 11, **dadurch gekennzeichnet, dass** der Wellradius (R) 10-12 mm beträgt, der Durchmesser (D) der Membran (9) 11 - 13 mm beträgt, die Einsatzwand oben außen 15 - 17 mm weit ist und die Abstandshalter (7, 8) eine Exzentrizität von über 1,3 mm des Zellkultureinsatzes (1) zum Well (2) gewährleisten.

14. Zellkultureinsatz nach Anspruch 1, **dadurch gekennzeichnet, dass** er aus eingefärbtem Material besteht.

15. Zellkultureinsatz nach Anspruch 14, **dadurch gekennzeichnet, dass** er aus transparentem Material besteht.

## Claims

1. Cell culture insert (1), comprising a beaker-shaped insert wall (11), a base (9) consisting of a membrane filter, an upper edge with support arms (3, 4) distributed around it, and lateral spacers (7, 8) for a vertical and horizontal orientation in a well (2) containing a liquid culture medium (N) in a cell culture plate, **characterized in that**
the spacers (7, 8) are distributed around the periphery of the cell culture insert (1) and have different radial lengths in such a way that a large feed opening (5) and several smaller openings (5A) are formed.

2. Cell culture insert according to claim 1, **characterized in that** at least two of the spacers (7) are triangular spacer ligaments, which are tapered towards the bottom.

3. Cell culture insert according to claim 1, **characterized in that** the shortest spacer (8) defines a minimum distance between the insert wall (11) of the cell culture insert (1) and an inner wall (W) of the well (2), which prevents a rise of the liquid culture medium (N) by capillary action.

4. Cell culture insert according to claim 1, **characterized in that** the distance between the insert wall (11) of the cell culture insert (1) and an inner wall (W) of the well (2) is defined by the shape of the insert wall (11), which is tapered towards the bottom.

5. Cell culture insert according to claim 1, **characterized in that** the cell culture insert (1) is hung and held in an eccentric position in the well (2) by three supporting arms (3, 4).

6. Cell culture insert according to claim 5, **characterized in that** the support arms (3, 4) are joined on top of the spacers (7, 8) and are arranged circumferentially, each at an angle of 120 degrees to the other.

7. Cell culture insert according to claim 1, **characterized in that** at the top of the insert wall (11), at least between the two longer support arms (4), a recess (12) is formed, with a lower edge (13) which is situated at a distance (10) above a normal level of the liquid culture medium (N).

8. Cell culture insert according to claim 7, **characterized in that** the lower edge (13) of the recess (12) in the wall is curved towards the support arms (3, 4).

9. Cell culture insert according to claim 7, **characterized in that** the recess in the wall has a depth (T), which is about 20% of an insert height (H) of the insert (1, 1 A).

10. Cell culture insert according to claim 1, **characterized in that** its insert wall (11) has a wall thickness (WU) which is greater at the membrane side than at the top.

11. Cell culture insert according to claim 10, **characterized in that** the outside of its insert wall (11) is tapered from top to bottom with a skew of about 1.5 degrees, and the inside of the insert wall (11) has a skew of 3.3 degrees.

12. Cell culture insert according to claim 11, **characterized in that** the diameter (D) of the membrane (9) is greater than an inner well radius (R) of the surrounding well (2).

13. Cell culture insert according to claim 11, **characterized in that** the well radius (R) is 10-12 mm, the diameter (D) of the membrane (9) is 11-13 mm, the outer top width of the insert wall is 15-17 mm, and the spacers (7, 8) guarantee an eccentricity of the cell culture insert (1) in relation to the well (2) of more than 1.3 mm.

14. Cell culture insert according to claim 1, **characterized in that** it is made of colored material.

15. Cell culture insert according to claim 14, **characterized in that** it is made of transparent material.

## Revendications

1. Insertion de culture cellulaire (1), comprenant une paroi d'insertion (11) en forme de gobelet, une base de filtre en membrane (9), un bord supérieur avec des bras de support (3, 4) répartis sur celui-ci, et des espaceurs latéraux (7, 8) de une orientation verticale et horizontale dans un godet (2) avec un milieu de culture liquide (N) dans une plaque de culture cellulaire, **caractérisée en ce que** les espaceurs latéraux (7, 8) sont répartis sur la périphérie de l'insertion de culture cellulaire (1) et qu'ils ont des longueurs radiales différentes, de telle manière qu'une grande fenêtre d'alimentation (5) et plusieurs petites fenêtres (5A) sont formés.

2. Insertion de culture cellulaire selon la revendication 1, **caractérisée en ce qu'**au moins deux espaceurs latéraux (7) sont des étançons triangulaires qui sont effilés vers le bas.

3. Insertion de culture cellulaire selon la revendication 1, **caractérisée en ce que** l'espaceur latéral (8) le plus court définit une distance minimale entre la paroi d'insertion (11) de l'insertion de culture cellulaire (1) et une paroi intérieure (W) du godet (2), alors le milieu de culture liquide (N) ne peut pas monter par action capillaire.

4. Insertion de culture cellulaire selon la revendication 1, **caractérisée en ce que** la distance entre la paroi d'insertion (11) de l'insertion de culture cellulaire (1) et une paroi intérieure (W) du godet (2) est définie par la forme de la paroi d'insertion (11), qui est effilée vers le bas.

5. Insertion de culture cellulaire selon la revendication 1, **caractérisée en ce que** l'insertion de culture cellulaire (1) est suspendue et maintenue dans une position excentrique dans le godet (2) par trois bras de support (3, 4).

6. Insertion de culture cellulaire selon la revendication 5, **caractérisée en ce que** les bras de support (3, 4) sont reliés au-dessus des espaceurs latéraux (7, 8) et sont disposés sur le périmètre, chacun décalé de 120 degrés.

7. Insertion de culture cellulaire selon la revendication 1, **caractérisée en ce qu'**un évidement (12) est formé dans la partie supérieure de la paroi d'insertion (11), au moins entre les deux bras de support (4) les plus longs, avec un bord inférieur (13) qui est situé à une distance (10) au dessus d'un niveau normal du milieu de culture liquide (N).

8. Insertion de culture cellulaire selon la revendication 7, **caractérisée en ce que** le bord inférieur (13) de l'évidement (12) dans la paroi est courbé vers les bras de support (3, 4).

9. Insertion de culture cellulaire selon la revendication 7, **caractérisée en ce que** l'évidement dans la paroi a une profondeur (T), qui mesure environ 20% d'une hauteur d'insertion (H) de l'insertion (1, 1 A).

10. Insertion de culture cellulaire selon la revendication 1, **caractérisée en ce que** l'épaisseur (WU) de sa paroi d'insertion (11) est plus grande au côté de la membrane qu'au côté supérieure.

11. Insertion de culture cellulaire selon la revendication 10, **caractérisée en ce que** l'extérieur de sa paroi d'insertion (11) s'effile de haut en bas avec une inclinaison d'environ 1,5 degrés, et l'intérieur de la paroi d'insertion (11) a une inclinaison de 3,3 degrés .

12. Insertion de culture cellulaire selon la revendication 11, **caractérisée en ce que** le diamètre (D) de la membrane (9) est plus long qu'un rayon intérieur (R) du godet (2) qui l'entoure.

13. Insertion de culture cellulaire selon la revendication 11, **caractérisée en ce que** le rayon (R) du godet est de 10-12 mm, le diamètre (D) de la membrane (9) est de 11-13 mm, la largeur extérieure supérieure de la paroi d'insertion est de 15 -17 mm, et les espaceurs latéraux (7, 8) assurent une excentricité de l'insertion de culture cellulaire (1) par rapport au godet (2) de plus de 1,3 mm.

14. Insertion de culture cellulaire selon la revendication 1, **caractérisée en ce qu'**elle est faite d'un matériel colorée.

15. Insertion de culture cellulaire selon la revendication 14, **caractérisée en ce qu'**elle est faite d'un matériel transparent.
